# EUROPEAN PATENT APPLICATION

(11) **EP 1 908 493 A1**
(43) Date of publication of application: **09.04.2008**
(21) Application number: 06121720.4
(22) Date of filing: 04.10.2006
(51) Int. Cl.: A61Q 5/12, A61K 8/41, A61K 8/89

(54) **Pulverized hair care treatment**

(71) Applicant: Wella Aktiengesellschaft, 64274 Darmstadt (DE)
(72) Inventor: Kolly Hernandez, Marylinde, 1762, Givisiez (CH); Moenks, Monika, 3185, Schmitten (CH)

(57) **Abstract**

The present invention relates to pulverized, non-fluid hair conditioning products comprising a fluid hair conditioning composition which is absorbed on a solid carrier, wherein the fluid hair conditioning composition contains at least one hair care agent, selected from the group consisting of hair conditioning surfactants, hair conditioning polymers, hair conditioning silicones, fatty alcohols, oils, panthenol, amino acids, panthenyl ethyl ether, sorbitol, betaine, creatine and protein hydrolysates; and wherein the carrier is a non-waxy material which is solid at room temperature. The products can be made by first dissolving a gas in said fluid hair conditioning composition at high pressure, then expanding the liquid/gas solution, wherein said solid carrier is added either before, or during or shortly after said expansion. The products can be used in a method of conditioning human hair.

## Description

### FIELD OF THE INVENTION

The present invention relates to pulverized, non-fluid hair conditioning products comprising a fluid hair conditioning composition which is absorbed on a solid, non-waxy carrier, wherein the fluid hair conditioning composition contains at least one hair care agent. The products can be made by pulverizing a fluid hair conditioning composition on a solid carrier, especially by first dissolving a gas in said fluid hair conditioning composition at high pressure, then expanding the liquid/gas solution, wherein the solid carrier is added either before, or during or shortly after the expansion.

### BACKGROUND OF THE INVENTION

Healthy, natural hair feels both firm and soft. It is easy to disentagle when wet or dry. When kept clean, it has a glossy, non-greasy look. Hair condition may be adversely affected due to physiological causes or by over-vigourous mechanical or chemical treatments. Such as e.g. bleaching, perming, washing with excessive detergents, too frequent or excessive brushing, hot blow drying etc. This may lead to hair that is dull-looking, brittle to the touch, has decreased combability, increased porosity, lower disruption point, decrease in sulphur content or degradation of polypeptide chains. Hair conditioning products are designed to treat and improve one or more of these negative hair conditions and to restore the hair's natural beauty, e.g. to give it lightness, volume, spring, control, suppleness, softness and sheen. The great majority of conventional conditiong formulations are aqueous cationic emulsions containing cationic conditiong agents (e.g. surfactants, polymers or silicones) and waxes (mainly fatty alcohols). They are typically applied after shampooing to the wet hair and are either rinsed off almost immediately, or left on the hair for a suitable residence time (e.g. 1 to 2 min) before rinsing. Deep conditioners such as masques or packs can also be left on the hair for a prolonged time to intensify the conditioning effect. Due to the different application purposes, conditioning demands and user preferences, there is a plentitude of different product types varying in product texture, rheology and active agent concentrations. Conventional hair conditioning products can be in the form of creams, cream gels, fluid or liquid emulsions, gel emulsions, lotions, liquid gels etc. They can be clear (e.g. micro emulsions, nano emulsions or lotions) or opacified (e.g. emulsified liquids or creams) and the texture can vary from liquid over slight gels to soft creams and thick creams. The relative amounts of cationic conditioning agents, waxes and, emulsifiers typically determines the appearance and the rheology of the product as well as the level of conditioning effect provided to the hair.

The demands vary depending on the nature, abundance and condition of the hair and on the habits and preferences of the user, e.g. the hair dresser or the consumer. With one given ready-to-use product only one or a very reduced number of demands can be satisfied. Therefore, a need exists to simplify and reduce the number of products and to provide products which satisfy a greater number of demands and which allow the user a greater flexibilty to treat different type of hairs, or to treat different parts of the hair differently or to satisfy different needs, habits or preferences (e.g. for product textures) of the user or her hair dresser. These benefits should be achieved without compromising the basic conditioning effect, i.e. providing almost the same level of conditioning as coventional ready-to-use products. It is one object of the invention to meet these needs.

### SUMMARY OF THE INVENTION

The present invention is directed to a pulverized, non-fluid hair conditioning product comprising a fluid hair conditioning composition which is absorbed on a solid carrier, wherein the fluid hair conditioning composition contains at least one hair care agent, selected from the group consisting of hair conditioning surfactants, hair conditioning polymers, hair conditioning silicones, fatty alcohols, oils, panthenol, amino acids, panthenyl ethyl ether, sorbitol, betaine, creatine and protein hydrolysates; and wherein said carrier is a non-waxy material which is solid at room temperature (25 °C). The pulverized, non-fluid hair conditioning product can be made by first dissolving a gas in said fluid hair conditioning composition at high pressure, then expanding the liquid/gas solution, wherein said solid carrier is added either before, or during or shortly after said expansion.

The present invention is further directed to methods of hair conditioning using the non-fluid hair conditioning product. These and other features, aspects, and advantages of the present invention will become evident to those skilled in the art from a reading of the present disclosure.

### DETAILED DESCRIPTION OF THE INVENTION

The pulverized, non-fluid hair conditioning products of the present invention are of a non-fluid consistency. They are preferably solid or semi-solid and comprise a fluid hair conditioning composition containing at least one hair care agent and wherein this fluid hair conditioning composition is absorbed on a solid carrier.

Each of the components, as well as preferred or optional components and the methods of making and using the product are described in detail hereinafter. All percentages, parts and ratios are based upon the total weight of the compositions of the present invention, unless otherwise specified. All such weights as they pertain to listed ingredients are based on the active level and therefore do not include solvents or byproducts that may be included in commercially available materials, unless otherwise specified. All molecular weights as used herein are weight average molecular weights expressed as grams/mole, unless otherwise specified.

Herein, "comprising" means that other steps and other ingredients which do not affect the end result can be added. This term encompasses the terms "consisting of" and "consisting essentially of". The compositions and methods of the present invention can comprise, consist of, and consist essentially of the essential elements and limitations of the invention described herein, as well as any of the additional or optional ingredients, components, steps, or limitations described herein.

By "hair" is meant preferably human scalp hair. The term "pulverized" as used herein, means a consistency which can be in the form of a powder consisting of a plurality of solid particles. The powder particles can be free flowing but can also be agglomerated due to stickiness giving the product a crumbly appearance. The term "non-fluid" as used herein, means compositions that are either in the form of a free flowing powder consisting of solid particles or in the form of a non-flowing agglomeration of particles. The non-fluid compositions preferably have a melting point above 25°C, more preferred above 100°C.

The term "fluid" as used herein, means either compositions that are non-viscous, i.e. have viscosities similar to water such as aqueous or aqueous-ethanolic lotions or compositions that are thickened with a gelifier up to viscosities of up to e.g. 100000 mPa s, as long as they are at least squeezable from tube packagings, i.e. flow under increased shear stress. The term "fluid" also comprises higher viscous consistencies such as e.g. soft creams, semi-solids or semi-solid waxes which are flowable at least under higher pressure of e.g. from 5 to 800 bar. All viscosities mentioned herein are measured as dynamic viscosity with a Haake VT-550 Rheometer, measurement body SV-DIN at a temperature of 25° C and at a shear rate of 50 s⁻¹, unless otherwise indicated. The term "non-waxy material" as used herein, means materials or substances that do not have the haptic and texture properties typical for a wax-like product such as paraffin wax (e.g. non-fluid and plastic at 20°C; softens or becomes fluid under shear or warming and melts between 25 and 100 °C without decomposition).

Hair conditioning materials or compositions are compounds or compositions which impart hair conditioning properties to hair (especially to human scalp hair), e.g. contribute in restoring the hair's natural beauty, increase the combability, the sheen, the suppleness, the softness, the lightness, the volume, the spring or the control of hair. In particular, hair conditioning agents are those cosmetic ingredients listed in the International Cosmetic Ingredient Dictionary and Handbook, 11th edition 2006 with the function "Hair Conditioning Agents".

All cited references are incorporated herein by reference in their entireties. Citation of any reference is not an admission regarding any determination as to its availability as prior art to the claimed invention.

### Fluid hair conditioning composition

The fluid hair conditioning composition that is absorbed on the solid carrier contains at least one hair conditioning agent which is dissolved or dispersed in a suitable cosmetically acceptable solvent. The fluid composition may be a solution or an emulsion. It may be thickened or gelled by suitable thickeners or gelling agents. The hair conditioning agents are selected from the group consisting of hair conditioning surfactants, hair conditioning polymers, hair conditioning silicones, hair conditioning amidoamines, panthenol, amino acids, betain and protein hydrolysates. The concentration of the conditioning agent in the fluid composition can be sufficient to provide the desired conditioning benefits, and as will be apparent to one of ordinary skill in the art. Such concentration can vary with the conditioning agent, the conditioning performance desired, the average size of the conditioning agent particles, the type and concentration of other components, and other like factors. The hair conditioning agents can be contained in the fluid hair conditioning composition in a quantity of e.g. from 0.01 to 20 wt. %, or particularly from 0.05 to 10 wt.%, or from 0.1 to 5 wt. %.

### Solvent

The preferred solvent is aqueous of aqueous-alcoholic. By "aqueous" it is meant that the compositions contain almost only water as solvent, i.e. organic solvents such as C1- to C4 alcohols are not present or they are present only in very minor amounts such as below 2 or below 1% by weight of the fluid composition. Deionized water is preferably used. Water from natural sources containing mineral cations can also be used, depending on the desired characteristic of the product. By "aqueous-alcoholic" it is meant that the compositions contain significant amounts of water as well as significant amounts of alcoholic solvents. Significant amounts are amounts of e.g. at least 5% by weight or more. The level and species of the solvents are selected according to the compatibility with other components, and other desired characteristics of the product. Alcoholic solvents are organic compounds which are liquid at room temperature (25°). The amount of alcohol is preferably 0 to 50% by weight, more preferably from 1 to 25% by weight of the liquid composition. Alcohols can be those conventionally used for cosmetic purposes, e.g. monohydric C1 to C6 alcohols such as ethanol and isopropanol. Ethanol is especially preferred. The water content is preferably from 40 to 95, more preferred from 50 to 90% by weight of the fluid composition. An aqueous-ethanolic carrier can contain for example 5 to 25% by weight ethanol and 60 to 80% by weight water, based on the total composition. The pH is preferably in the range of from 2 to 8, more preferably from 2,5 to 6,5. Buffers and other pH adjusting agents can be included to achieve or stabilize the desirable pH.

### Hair conditioning surfactants

The hair conditioning agent can be a hair conditioning surfactant. Preferred are cationic surfactants, amino surfactants and amidoamine compounds. Suitable cationic surfactants or amino surfactants contain amino groups and/or quaternized hydrophilic ammonium groups, which carry a positive charge in aqueous solution and which can be represented by the general formula

N⁽⁺⁾R¹R²R³R⁴ X⁽⁻⁾

wherein R1 to R4 independently from one another stand for aliphatic groups, aromatic groups, alkoxy groups, polyoxyalkylene groups, alkylamido groups, hydroxyalkyl groups, aryl groups or alkaryl groups with 1 to 22 C atoms, wherein at least one residue has at least 6, preferably at least 8 C atoms and X⁻ represents an anion, for example, a halide, acetate, phosphate, nitrate or alkyl sulfate, preferably a chloride. In addition to the carbon atoms and the hydrogen atoms, the aliphatic groups can also contain cross-compounds, or other groups, such as, for example, additional amino groups. Examples of suitable cationic surfactants are the chlorides or bromides of alkyldimethylbenzylammonium salts, alkyltrimethylammonium salts, e.g. cetyltrimethylammonium chloride or bromide, tetradecyltrimethylammonium chloride or bromide, alkyldimethylhydroxyethylammonium chlorides or bromides, dialkyldimethylammonium chlorides or bromides, alkylpyridinium salts, for example lauryl- or cetylpyridinium chloride, alkylamidoethyltrimethylammonium ether sulfates as well as compounds with cationic character such as amine oxides, e.g. alkylmethylamine oxides or alkylaminoethyldimethylamine oxides. Especially preferred are C8-22 alkyldimethylbenzylammonium compounds, C8-22 alkyltrimethylammonium compounds, especially cetyltrimethylammonium chloride, C8-22 alkyldimethylhydroxyethylammonium compounds, di-(C8-22 alkyl)-dimethylammonium compounds, C8-22 alkylpyridinium salts, C8-22 alkylamidoethyltrimethylammonium ether sulfates, C8-22 alkylmethylamine oxides, and C8-22 alkylaminoethyldimethylamine oxides.

In addition to the aforementioned cationic surfactants, other suitable cationic or amino-substituted surfactants are those of the formula R1-NH-(CH₂)n-NR2R3 or of the formula R1-NH-(CH₂)n-N⁺R2R3R4 X⁻
wherein R1 is an acyl or an alkyl residue with 8 to 24 C atoms, which can be branched or linear, saturated or unsaturated, whereby the acyl and/or the alkyl residue can contain one or more OH groups, R2, R3 and R4 independently of one another are hydrogen, alkyl or alkoxyalkyl residues with 1 to 6 C atoms, which can be the same or different, saturated or unsaturated and can be substituted with one or more hydroxy groups, X⁻ is an anion, especially a halide ion or a compound of the general formula RSO₃⁻, wherein R has the meaning of saturated or unsaturated alkyl residues with 1 to 4 C atoms, and n means a whole number between 1 and 10, preferably from 2 to 5.

The active hair-conditioning compound can also be an amidoamine and/or a quaternized amidoamine of the aforementioned formulae, wherein R1 is a branched or linear, saturated or unsaturated acyl residue with 8 to 24 C atoms that can contain at least one OH group. Preferred are such amines and/or quaternized amines, in which at least one of the residues R2, R3 and R4 means a residue according to the general formula CH₂CH₂OR5, wherein R5 can have the meaning of alkyl residues with 1 to 4 C atoms, hydroxyethyl or H. Suitable amines or amidoamines, which can be optionally quaternized, are especially such with the INCI names Ricinoleamidopropyl Betaine, Ricinoleamidopropyl Dimethylamine, Ricinoleamidopropyl Dimethyl Lactate, Ricinoleamidopropyl Ethyldimonium Ethosulfate, Ricinoleamidopropyltrimonium Chloride, Ricinoleamidopropyltrimonium Methosulfate, Cocamidopropyl Betaine, Cocamidopropyl Dimethylamine, Cocamidopropyl Ethyldimonium Ethosulfate, Cocamidopropyltrimonium Chloride, Behenamidopropyl Dimethylamine, Isostearylamidopropyl Dimethylamine, Stearylamidopropyl Dimethylamine, Quaternium-33, Undecyleneamidopropyltrimonium Methosulfate.

### Hair conditioning polymers

The hair conditioning agent can be a hair conditioning polymer. Preferred are polymers which contain quaternized hydrophilic ammonium groups or which contain amino groups which can carry a positive charge by protonation in aqueous solution. The hair conditioning polymer can be cationic, amphoteric or zwitterionic. It can be synthetic or natural. The term "natural polymer" also comprises chemically modified polymers of natural origin. Preferred are polymers which are soluble in an aqueous or aqueous-alcoholic solvent.

The compositions of the present invention can comprise cationic polymer. When included, concentrations of the cationic polymer in the composition can typically range from 0.05% to 3%, preferably from 0.075% to 2.0%, more preferably from 0.1% to 1.0%. Preferred cationic polymers will have cationic charge densities of at least 0.9 meq/gm, preferably at least 1.2 meq/gm, more preferably at least 1.5 meq/gm, but also preferably less than 7 meq/gm, more preferably less than 5 meq/gm, at the pH of intended use of the composition, which pH will generally range from about pH 3 to about pH 9, preferably between about pH 4 and about pH 8. Herein, "cationic charge density" of a polymer refers to the ratio of the number of positive charges on the polymer to the molecular weight of the polymer. The average molecular weight of such suitable cationic polymers can be between e.g. 10,000 and 10 million, preferably between 50,000 and 5 million, more preferably between 100,000 and 3 million.
Suitable cationic polymers for use in the compositions of the present invention contain cationic nitrogen-containing moieties such as quaternary ammonium or cationic protonated amino moieties. The cationic protonated amines can be primary, secondary, or tertiary amines (preferably secondary or tertiary), depending upon the particular species and the selected pH of the composition. Any anionic counterions can be used in association with the cationic polymers so long as the polymers remain soluble in water, in the composition, or in a coacervate phase of the composition, and so long as the counterions are physically and chemically compatible with the essential components of the composition or do not otherwise unduly impair product performance, stability or aesthetics. Non limiting examples of such counterions include halides (e.g., chloride, fluoride, bromide, iodide), sulfate and methylsulfate. Non limiting examples of such polymers are described in the CTFA.

Suitable synthetic cationic polymers are homo- or copolymers consisting of at least one of the following monomers: dialkylaminoalkyl acrylate, dialkylaminoalkyl methacrylate, monoalkylaminoalkyl acrylate, and monoalkyl aminoalkyl methacrylate, trialkyl methacryloxyalkyl ammonium, trialkyl acryloxyalkyl ammonium, dialkyl diallyl ammonium, and quaternary vinyl ammonium monomers with cyclic groups containing cationic nitrogens.

Suitable cationic polymers preferably contain quaternary amino groups. Cationic polymers can be homo- or copolymers, where the quaternary nitrogen groups are contained either in the polymer chain or preferably as substituents on one or more of the monomers. The monomers containing ammonium groups can be copolymerized with non-cationic monomers. Suitable cationic monomer are unsaturated compounds that can undergo radical polymerization, which bear at least one cationic group, especially ammonium-substituted vinyl monomers such as, for example, trialkylmethacryloxy-alkylammonium, trialkylacryloxyalkylammonium, dialkyldiallylammonium and quaternary vinylammonium monomers with cyclic, cationic nitrogen-containing groups such as pyridinium, imidazolium or quaternary pyrrolidones, e.g. alkylvinylimidazolium, alkylvinylpyridinium, or alkylvinylpyrrolidone salts. The alkyl groups of these monomers are preferably lower alkyl groups such as, for example, C1 to C7 alkyl groups, and especially preferred are C1 to C3 alkyl groups.

Preferred cationic substituted monomers are the cationic substituted dialkylaminoalkyl acrylamides, dialkylaminoalkyl methacrylamides, and combinations thereof. These preferred monomers conform the to the formula: 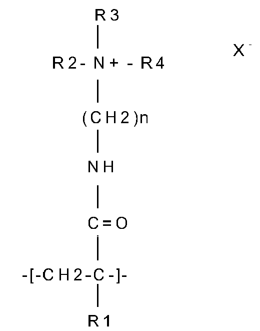 wherein R¹ is hydrogen, methyl or ethyl; each of R², R³ and R⁴ are independently hydrogen or a short chain alkyl having from about 1 to about 8 carbon atoms, preferably from about 1 to about 5 carbon atoms, more preferably from about 1 to about 2 carbon atoms; n is an integer having a value of from about 1 to about 8, preferably from about 1 to about 4; and X is a counterion. The nitrogen attached to R², R³ and R⁴ may be a protonated amine (primary, secondary or tertiary), but is preferably a quaternary ammonium wherein each of R², R³ and R⁴ are alkyl groups a non limiting example of which is polymethyacrylamidopropyl trimonium chloride, available under the trade name Polycare 133, from Rhone-Poulenc, Cranberry, N.J., U.S.A.

The monomers containing ammonium groups can be copolymerized with non-cationic monomers. Suitable comonomers are, for example, acrylamide, methacrylamide, alkyl- and dialkylacrylamide, alkyl- and dialkylmethacrylamide, alkyl acrylate, alkyl methacrylate, vinylcaprolactone, vinylcaprolactam, vinylpyrrolidone, vinyl esters, for example vinyl acetate, vinyl alcohol, propylene glycol or ethylene glycol, wherein the alkyl groups of these monomers are preferably C1 to C7 alkyl groups, and especially preferred are C1 to C3 alkyl groups.

Suitable polymers with quaternary amino groups are, for example, those described in the CTFA Cosmetic Ingredient Dictionary under the designations Polyquaternium such as methylvinylimidazolium chloride/vinylpyrrolidone copolymer (Polyquaternium-16) or quaternized vinylpyrrolidone/dimethylaminoethyl methacrylate copolymer (Polyquaternium-11).

Preferred cationic polymers of synthetic origin: cationic diallyl quaternary ammonium-containing polymers, including, for example, dimethyldiallylammonium chloride homopolymer, copolymers of acrylamide and dimethyldiallylammonium chloride (referred to in the industry by CTFA as Polyquaternium 6 and Polyquaternium 7, respectively); quaternary ammonium polymers, formed by the reaction of diethyl sulfate with a copolymer from vinylpyrrolidone and dimethylaminoethyl methacrylate, especially vinylpyrrolidone/dimethylaminoethyl methacrylate methosulfate copolymer (polyquaternium-11, e.g. Gafquat® 755 N, Gafquat® 734); copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16, e.g. LUVIQUAT® HM 550); Polyquaternium-35; Polyquaternium-57; polymers from trimethylammonium ethyl methacrylate chloride; terpolymers from dimethyldiallyl ammonium chloride, sodium acrylate and acrylamide (e.g. Merquat® Plus 3300); copolymers from vinylpyrrolidone, dimethylaminopropyl methacrylamide and methacryloylaminopropyllauryldimethylammonium chloride; terpolymers from vinylpyrrolidone, dimethylaminoethyl methacrylate and vinylcaprolactam (e.g. Gaffix® VC 713); vinylpyrrolidone/methacrylamidopropyltrimethylammonium chloride copolymers (e.g. Gafquat® HS 100); copolymers from vinylpyrrolidone and dimethylaminoethyl methacrylate; copolymers from vinylpyrrolidone, vinylcaprolactam and dimethylaminopropylacrylamide; poly-or oligoesters formed from at least one first type of monomer, that is selected from hydroxyacids substituted with at least one quaternary ammonium group.

Suitable cationic polymers that are derived from natural polymers are especially cationic derivatives of polysaccharides, for example, cationic derivatives of cellulose, starch or guar. Furthermore, chitosan and chitosan derivatives are also suitable. Cationic polysaccharides are, for example, represented by the general formula

G-O-B-N⁺R^{a}R^{b}R^{c} X⁻

G is an anhydroglucose residual group, e.g. starch or cellulose anhydroglucose;
B is a divalent linking group, for example alkylene, oxyalkylene, polyoxyalkylene or hydroxyalkylene, or combination thereof;
R^{a}, R^{b}, and R^{c}, independently from one another, are alkyl, aryl, alkylaryl, arylalkyl, alkoxyalkyl, or alkoxyaryl, any of which can have up to 18 C atoms, wherein the total number of C atoms in R^{a}, R^{b}, and R^{c} is preferably a maximum of 20;
X is a conventional counter-anion, for example, a halide, acetate, phosphate, nitrate, or alkyl sulfate, preferably a chloride.

Preferred cationic cellulose polymers are salts of hydroxyethyl cellulose reacted with trimethyl ammonium substituted epoxide, referred to in the industry (CTFA) as Polyquaternium 10 and available from Amerchol Corp. (Edison, N.J., USA) in their Polymer LR, JR, and KG series of polymers. Other suitable types of cationic cellulose includes the polymeric quaternary ammonium salts of hydroxyethyl cellulose reacted with lauryl dimethyl ammonium-substituted epoxide referred to in the industry (CTFA) as Polyquaternium 24. These materials are available from Amerchol Corp. under the tradename Polymer LM-200. Other cationic celluloses are, for example, those with the INCI name Polyquaternium-4. Other suitable cationic polymers include cationic guar gum derivatives, such as guar hydroxypropyltrimonium chloride, specific examples of which include the Jaguar series commercially avaialable from Rhone-Poulenc Incorporated and the N-Hance series commercially available from Aqualon Division of Hercules, Inc.

Especially preferred cationically-active substances are chitosan, chitosan salts and chitosan derivatives. Chitosans that can be used according to the invention can be fully or partially deacetylated chitins. By way of example, the molecular weight can be distributed over a broad range, from 20,000 to about 5 million g/mol, for example from 30,000 to 70,000 g/mol. However, the molecular weight will preferably lie above 100,000 g/mol, and especially preferred from 200,000 to 700,000 g/mol. The degree of deacetylation is preferably from 10 to 99%, and especially preferably from 60 to 99%. A preferred chitosan salt is chitosonium pyrrolidone carboxylate, e.g. Kytamer® PC with a molecular weight of from about 200,000 to 300,000 g/mol and a degree of deacetylation of from 70 to 85%. Chitosan derivatives that can be considered include quaternized, alkylated or hydroxyalkylated derivatives, e.g. hydroxyethyl, hydroxypropyl or hydroxybutyl chitosan. The chitosans or chitosan derivatives are preferably present in their neutralized or partially neutralized form. The degree of neutralization will be preferably at least 50%, especially preferably between 70 and 100%, as calculated on the basis of the number of free base groups. For the neutralization agent, in principle any cosmetically compatible inorganic or organic acids can be used such as, for example, formic acid, tartaric acid, malic acid, lactic acid, citric acid, pyrrolidone carboxylic acid, hydrochloric acid and others, of which pyrrolidone carboxylic acid is especially preferred.

Preferred cationic polymers derived from natural sources:
cationic cellulose derivatives from hydroxyethyl cellulose and diallyldimethyl ammonium chloride; cationic cellulose deviates from hydroxyethyl cellulose and trimethylammonium-substituted epoxide; chitosan and its salts; hydroxyalkyl chitosans and their salts; alkylhydroxyalkyl chitosans and their salts; N-hydroxyalkylchitosan alkyl ethers.

Suitable synthetic, amphoteric hair conditioning polymers are polymers with anionic or acidic functional groups as well as cationic or basic functional groups. The acidic or anionic functional groups can be e.g. carboxylic acid groups or sulphonic acid groups. Cationic or basic functional groups are in particular primary, secondary or tertiary amine groups or quaternary ammonium groups. Examples for hair conditioning polymers are terpolymer of acrylic acid, methyl acrylate and methacrylamidopropyl trimethylammonium chloride (INCI-name: polyquaternium-47); copolymer of acrylamidopropyl trimethylammonium chloride and acrylates; or copolymers of acrylamide, acrylamidopropyl trimethylammonium chloride, 2-amidopropyl acrylamide sulfonate and dimethylaminopropyl amine (INCI-name: polyquaternium-43); copolymers of acrylic acid and dimethyldiallylammonium chloride (INCI-name polyquaternium-22), terpolymers of acrylic acid with dimethyldiallylammonium chloride and acrylamide (INCI name polyquaternium-39). Suitable are also polymers with betaine groups, e.g. copolymers of methacryloyl ethylbetaine and two or more monomers selected from acrylic acid and its alkyl esters (INCI-name Methacryloyl Ethyl Betaine/Acrylates Copolymer).

### Hair conditioning silicones

The hair conditioning agent can be a hair conditioning silicone, e.g., silicone oil, amino silicone, cationic silicone, silicone gum, high refractive silicone, or silicone resin. The silicone compounds include, in particular, the materials with the INCI designations Cyclomethicone, Dimethicone, Dimethiconol, Dimethicone Copolyol, Phenyl Trimethicone, Amodimethicone, Trimethylsilylamodimethicone, Stearyl Siloxysilicate, Polymethylsilsesquioxane, and Dimethicone Crosspolymer. Silicone resins and silicone elastomers are also suitable, wherein these are highly crosslinked siloxanes. Crosslinked silicones can be used simultaneously to provide a suitable consistency to the composition. Preferred silicones are: cyclic dimethyl siloxanes, linear polydimethyl siloxanes, block polymers from polydimethyl siloxane and polyethylene oxide and/or polypropylene oxide, polydimethyl siloxanes with terminal or lateral polyethylene oxide or polypropylenoxide radicals, polydimethyl siloxanes with terminal hydroxyl groups, phenyl-substituted polydimethyl siloxanes, silicone emulsions, silicone elastomers, silicone waxes, silicone gums, amino-substituted silicones, silicones substituted with quaternary ammonia groups, and crosslinked silicones.

The concentration of the silicone conditioning agent typically ranges from about 0.01 % to about 10%, preferably from about 0.1 % to about 8%, more preferably from about 0.1% to about 5%, more preferably from about 0.2% to about 3%. The silicone compounds include volatile and nonvolatile silicones. Preferred are non-volatile silicone conditioning agents. If volatile silicones are present, it will typically be incidental to their use as a solvent or carrier for commercially available forms of non-volatile silicone materials ingredients, such as silicone gums and resins. The silicones may be soluble or insoluble in the fluid composition. Preferred are insoluble silicones which may be dispersed in the fluid. The dispersed silicone particles typically have a number average particle diameter ranging from about 0.01µm to about 50µm. For small particle application to hair, the number average particle diameters typically range from about 0.01µm to about 4µm, preferably from about 0.01µm to about 2µm, more preferably from about 0.01µm to about 0.5µm. For larger particle application to hair, the number average particle diameters typically range from about 4µm to about 50µm, preferably from about 6µm to about 30µm, more preferably from about 9µm to about 20µm, more preferably from about 12µm to about 18µm. The silicone conditioning agents for use in the compositions of the present invention preferably have a viscosity, as measured at 25°C, from 20 to 2,000,000 mPa s, more preferably from 1,000 to 1,800,000 mPa s, or from 10,000 to 1,700,000 mPa s, or from 50,000 to 1,600,000 mPa s, more preferably from 100,000 to 1,500,000 mPa s.

### a. Silicone oils

Silicone fluids include silicone oils, which are flowable silicone materials having a viscosity, as measured at 25°C, less than 1,000,000 mPa s, preferably from 5 mP as to 1,000,000 mPa s, more preferably from 100 mPa s to 600,000 mPa s. Suitable silicone oils for use in the compositions of the present invention include polyalkyl siloxanes, polyaryl siloxanes, polyalkylaryl siloxanes, polyether siloxane copolymers, and mixtures thereof. Other insoluble, non-volatile silicone fluids having hair conditioning properties may also be used. Silicone oils include polyalkyl or polyaryl siloxanes which conform to the following Formula

R₃Si-O-(SiR2-O)ₓ-SiR₃

wherein R is aliphatic, preferably alkyl or alkenyl, or aryl, R can be substituted or unsubstituted, and x is an integer from 1 to about 8,000. Suitable R groups for use in the compositions of the present invention include, but are not limited to: alkoxy, aryloxy, alkaryl, arylalkyl, arylalkenyl, alkamino, and ether-substituted, hydroxyl-substituted, and halogen-substituted aliphatic and aryl groups. Suitable R groups also include cationic amines and quaternary ammonium groups. Preferred alkyl and alkenyl substituents are C₁ to C₅ alkyls and alkenyls, more preferably from C₁ to C₄, more preferably from C₁ to C₂. The aliphatic portions of other alkyl-, alkenyl-, or alkynyl-containing groups (such as alkoxy, alkaryl, and alkamino) can be straight or branched chains, and are preferably from C₁ to C₅, more preferably from C₁ to C₄, even more preferably from C₁ to C₃, more preferably from C₁ to C₂. As discussed above, the R substituents can also contain amino functionalities (e.g. alkamino groups), which can be primary, secondary or tertiary amines or quaternary ammonium. These include mono-, di- and tri- alkylamino and alkoxyamino groups, wherein the aliphatic portion chain length is preferably as described herein.

### b. Amino and Cationic silicones

Cation-active silicone compounds are also preferred. Suitable cation-active silicone compounds either have at least one amino group or at least one quaternary ammonium group. Silicone polymers with amino groups are known under the INCI designations Amodimethicone and Trimethylsiloxyamodimethicone. These polymers are polydimethylsiloxanes with aminoalkyl groups. The aminoalkyl groups can be lateral or terminal. Suitable amino silicones are as those of the general formula

(R₁)ₐG₃₋ₐ-Si-(-OSiG₂)ₙ-(-OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 0; b is 0 or 1, preferably 1; n is a number from 0 to 1,999, preferably from 49 to 499; m is an integer from 1 to 2,000, preferably from 1 to 10; the sum of n and m is a number from 1 to 2,000, preferably from 50 to 500; R₁ is a monovalent radical conforming to the general formula CqH_{2q}L, wherein q is an integer having a value from 2 to 8 and L is selected from the following groups:
- N(R₂)CH₂-CH₂-N(R₂)₂
- N(R₂)₂
- N(R₂)₃A⁻
- N(R₂)CH₂-CH₂-NR₂H₂A⁻
wherein R₂ is hydrogen, phenyl, benzyl, or a saturated hydrocarbon radical, preferably an alkyl radical from about C₁ to about C₂₀, and A⁻ is a halide ion.

A preferred amino silicone corresponding to the above formula is the polymer known as amodimethicone, which is shown in the following formula: 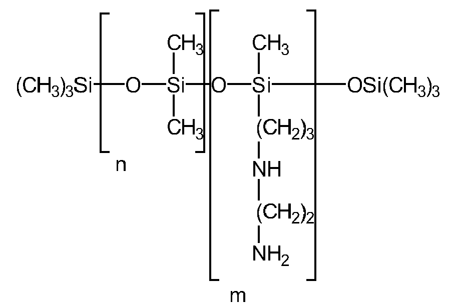 with n and m being numbers as described above.

Another preferred amino silicone is the polymer known as trimethylsiloxyamodimethicone, which is shown in the following formula:

Me₃SiO-(SiMe₂O-)ₙ-(Si(-OSiMe₃)(-(CH₂)₃-NH-(CH₂)₂-NH₂)-O-)ₘ-SiMe₃

with n and m being numbers as described above.

The molecular weight of the amino silicones is preferably between 500 and 100,000. The amine portion (meq/g) preferably ranges between 0.05 to 2.3, with 0.1 to 0.5 being particularly preferred.

Other cationic silicone which may be used in the compositions of the present invention are represented by the following formula: 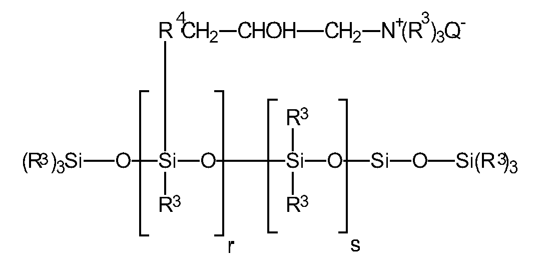 wherein R³ is a monovalent hydrocarbon radical from C₁ to C₁₈, preferably an alkyl or alkenyl radical, such as methyl; R₄ is a hydrocarbon radical, preferably a C₁ to C₁₈ alkylene radical or a C₁₀ to C₁₈ alkyleneoxy radical, more preferably a C₁ to C₈ alkyleneoxy radical; Q⁻ is a halide ion, preferably chloride; r is an average statistical value from 2 to 20, preferably from 2 to 8; s is an average statistical value from 20 to 200, preferably from 20 to 50. A preferred polymer of this class is known as UCARE SILICONE ALE 56™, available from Union Carbide.

Suitable cationic silicone polymers with two terminal quaternary ammonium groups are known under the INCI designation Quaternium-80. These are dimethylpolysiloxanes with 2 terminal alkyl ammonium groups. Suitable quaternary amino silicones are those of the general formula

(R²)₃N⁺-A-SiR₂-(OSi(R¹)₂)ₙ-OSiR₂-A-N⁺(R²)₃ 2X⁻

A stands for a divalent C1 to C20 alkylene compound group, which can also contain O and N atoms as well as OH groups and is preferably -(CH₂)₃OCH₂CHOHCH₂;
R independently are the same or different and mean C1 to C10 alkyl, phenyl, hydroxy, hydrogen, C1 to C10 alkoxy or acetoxy, or preferably C1-C4 alkyl, especially methyl;
R¹ independently are the same or different and mean hydrogen, C1 to C20 hydrocarbon, which can contain O and N atoms, or preferably C1 to C10 alkyl or phenyl, or especially preferably C1 to C4 alkyl, but particularly methyl;
R² independently mean C1 to C22 alkyl groups, which can contain hydroxyl groups and wherein preferably at least one of the groups has at least 10 C atoms and the remaining groups have 1 to 4 C atoms; n is a number of from 0 to 200, or preferably 10 to 100;
X is a halide ion, preferably chloride. These types of diquaternary polydimethylsiloxanes are available from GOLDSCHMIDT under the trade names Abil® Quat 3270, 3272, and 3274.

### c. Silicone gums

Other silicones suitable for use in the compositions of the present invention are the insoluble silicone gums. These gums are polyorganosiloxane materials having a viscosity, as measured at 25°C, of greater than or equal to 1,000,000 mPa s. Silicone gums are available for example from General Electric as SE 30, SE 33, SE 54 and SE 76. Specific non-limiting examples of silicone gums for use in the compositions of the present invention include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

### d. High refractive index silicones

Other non-volatile, insoluble silicone fluid conditioning agents that are suitable for use in the compositions of the present invention are those known as "high refractive index silicones", having a refractive index of at least about 1.46, preferably at least about 1.48, more preferably at least about 1.52, more preferably at least about 1.55. The refractive index of the polysiloxane fluid will generally be less than about 1.70, typically less than about 1.60. In this context, polysiloxane "fluid" includes oils as well as gums. The high refractive index polysiloxane fluid includes those represented by the general formula for the silicone oils above, as well as cyclic polysiloxanes such as those represented by the following formula: 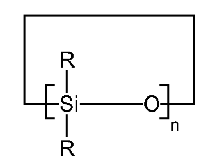 wherein R is phenyl or phenyl derivative (more preferably phenyl), alkyl, preferably C₁-C₄ alkyl (more preferably methyl), hydroxy, C₁-C₄ alkylamino (especially -R¹NHR²NH2 wherein each R¹ and R² independently is a C₁-C₃ alkyl, alkenyl, and/or alkoxy); and n is a number from about 3 to about 7, preferably from about 3 to about 5. The high refractive index polysiloxane fluids contain an amount of aryl-containing R substituents sufficient to increase the refractive index to the desired level, which is described herein. Additionally, R and n must be selected so that the material is non-volatile.
Aryl-containing substituents include those which contain alicyclic and heterocyclic five and six member aryl rings and those which contain fused five or six member rings. The aryl rings themselves can be substituted or unsubstituted. Generally, the high refractive index polysiloxane fluids will have a degree of aryl-containing substituents of at least about 15%, preferably at least about 20%, more preferably at least about 25%, even more preferably at least about 35%, more preferably at least about 50%. Typically, the degree of aryl substitution will be less than about 90%, more generally less than about 85%, preferably from about 55% to about 80%. Preferred high refractive index polysiloxane fluids have a combination of phenyl or phenyl derivative substituents (more preferably phenyl), with alkyl substituents, preferably C₁-C₄ alkyl (more preferably methyl), hydroxy, or C₁-C₄ alkylamino (especially -R'NHR²NH2 wherein each R¹ and R² independently is a C₁-C₃ alkyl, alkenyl, and/or alkoxy). When high refractive index silicones are used in the compositions of the present invention, they are preferably used in solution with a spreading agent, such as a silicone resin or a surfactant, to reduce the surface tension by a sufficient amount to enhance spreading and thereby enhance the glossiness (subsequent to drying) of hair treated with the compositions.

### e. Silicone resins

Other silicone conditioning agents are silicone resins. These resins are highly cross-linked polymeric siloxane systems. The cross-linking is introduced through the incorporation of trifunctional and tetrafunctional silanes with monofunctional or difunctional, or both, silanes during manufacture of the silicone resin. Silicone materials and silicone resins in particular, can conveniently be identified according to a shorthand nomenclature system known to those of ordinary skill in the art as "MDTQ" nomenclature. Under this system, the silicone is described according to presence of various siloxane monomer units which make up the silicone. Briefly, the symbol M denotes the monofunctional unit (CH₃)₃SiO_{0.5}; D denotes the difunctional unit (CH₃)₂SiO; T denotes the trifunctional unit (CH₃)SiO_{1.5}; and Q denotes the quadra- or tetra-functional unit SiO₂. Primes of the unit symbols (e.g. M', D', T', and Q') denote substituents other than methyl, and must be specifically defined for each occurrence. Preferred silicone resins for use in the compositions of the present invention include, but are not limited to MQ, MT, MTQ, MDT and MDTQ resins. Methyl is a preferred silicone substituent. Especially preferred silicone resins are MQ resins, wherein the M:Q ratio is from about 0.5:1.0 to about 1.5:1.0 and the average molecular weight of the silicone resin is from about 1000 to about 10,000. Typical cross-linked silicones are those with an INCI-designation which includes the term "crosspolymer".

### Fatty alcohols

The hair conditioning agent can be a fatty alcohol. The fatty alcohols can be saturated, mono-or poly-unsaturated, branched or unbranched and can have from 6 to 30, or preferably from 10 to 22, and most preferred from 12 to 22 carbon atoms. For example, decanol, octanol, octenol, dodecanol, dodecenol, decenol, octadienol, dodecadienol, decadienol, oleyl alcohol, eruca alcohol, ricinol alcohol, stearyl alcohol, isostearyl alcohol, cetyl alcohol, lauryl alcohol, myristyl alcohol, arachidyl alcohol, capryl alcohol, caprine alcohol, linoleyl alcohol, linolenyl alcohol, and behenyl alcohol can be used in terms of the invention, wherein this list should be considered exemplary and not limiting. The fatty alcohols are preferably derived, however, from natural fatty acids, wherein one can assume a recovery from the esters of fatty acids via reduction. Fatty alcohol portions, which are created by the reduction of naturally occurring triglycerides such as beef tallow, palm oil, peanut oil, turnip oil, cottonseed oil, soy oil, sunflower seed oil, and linseed oil or of their transesterification products with fatty acid esters occurring with the corresponding alcohols can be used according to the invention and thus represent a mixture of different fatty alcohols. Wool wax alcohols can also be used according to the invention.

### Oils

The hair conditioning agent can be an organic conditioning oil, either alone or in combination with other conditioning agents as described herein. Suitable hair-conditioning oils are, in particular, hydrophobic oils having a melting point of less than 25°C and a boiling point of preferably greater than 250°C, or particularly greater than 300°C. Volatile oils can also be used. In principle, any oil generally known to a person skilled in the art can be used. Suitable oils are e.g. hydrocarbon oils, liquid polyolefins and liquid fatty esters.

### a. Hydrocarbon oils

Suitable organic conditioning oils for use as conditioning agents in the compositions of the present invention include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from about C₁₂ to about C₁₉. Branched chain hydrocarbon oils, including hydrocarbon polymers, typically will contain more than 19 carbon atoms. Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from Amoco Chemical Corporation. The concentration of such hydrocarbon oils in the composition preferably range from about 0.05% to about 20%, more preferably from about 0.08% to about 1.5%, and even more preferably from about 0.1% to about 1%.

### b. Polyolefins

Organic conditioning oils for use in the compositions of the present invention can also include liquid polyolefins, more preferably liquid poly-α-olefins, more preferably hydrogenated liquid poly-α-olefins. Polyolefins for use herein are prepared by polymerization of C₄ to about C₁₄ olefenic monomers, preferably from about C₆ to about C₁₂. Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. Also suitable for preparing the polyolefin liquids are olefin-containing refinery feedstocks or effluents. Preferred hydrogenated α-olefin monomers include, but are not limited to: 1-hexene to 1-hexadecenes, 1-octene to 1-tetradecene, and mixtures thereof.

### c. Fatty Esters

Other suitable organic conditioning oils for use as the conditioning agent in the compositions of the present invention include, but are not limited to, fatty esters having at least 10 carbon atoms. These fatty esters include esters with hydrocarbyl chains derived from fatty acids or alcohols (e.g. mono-esters, polyhydric alcohol esters, and di- and tricarboxylic acid esters). The hydrocarbyl radicals of the fatty esters hereof may include or have covalently bonded thereto other compatible functionalities, such as amides and alkoxy moieties (e.g., ethoxy or ether linkages, etc.). Specific examples of preferred fatty esters include, but are not limited to: isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.
Other fatty esters suitable for use in the compositions of the present invention are mono-carboxylic acid esters of the general formula R'COOR, wherein R' and R are alkyl or alkenyl radicals, and the sum of carbon atoms in R' and R is at least 10, preferably at least 22. Still other fatty esters suitable for use in the compositions of the present invention are di- and tri-alkyl and alkenyl esters of carboxylic acids, such as esters of C₄ to C₈ dicarboxylic acids (e.g. C₁ to C₂₂ esters, preferably C₁ to C₆, of succinic acid, glutaric acid, and adipic acid). Specific non-limiting examples of di- and tri- alkyl and alkenyl esters of carboxylic acids include isocetyl stearyol stearate, diisopropyl adipate, and tristearyl citrate.
Other fatty esters suitable for use in the compositions of the present invention are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.
Still other fatty esters suitable for use in the compositions of the present invention are glycerides, including, but not limited to, mono-, di-, and tri-glycerides, preferably di-and tri-glycerides, more preferably triglycerides. For use in the compositions described herein, the glycerides are preferably the mono-, di-, and tri-esters of glycerol and long chain carboxylic acids, such as C₁₀ to C₂₂ carboxylic acids. A variety of these types of materials can be obtained from vegetable and animal fats and oils, such as sunflower seed oil, coconut oil, jojoba oil, castor oil, safflower oil, sesame oil, walnut oil, peach seed oil, tea tree oil, camellia oil, evening primrose oil, rice bran oil, mango seed oil, cuckoo flower oil, thistle oil, macadamia nut oil, grapeseed oil, apricot seed oil, babassu oil, Kukui nut oil, (sweet) almond oil, cottonseed oil, corn oil, olive oil, cod liver oil, avocado oil, palm oil, lanolin oil, wheat germ oil, pumpkin seed oil, mallow oil, hazelnut oil, canola oil, sasanqua oil and soybean oil. Synthetic oils include, but are not limited to, triolein and tristearin glyceryl dilaurate.
Other fatty esters suitable for use in the compositions of the present invention are water insoluble synthetic fatty esters. Some preferred synthetic esters conform to the following formula: 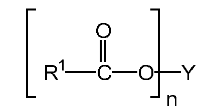 wherein R¹ is a C₇ to C₉ alkyl, alkenyl, hydroxyalkyl or hydroxyalkenyl group, preferably a saturated alkyl group, more preferably a saturated, linear, alkyl group; n is a positive integer having a value from 2 to 4, preferably 3; and Y is an alkyl, alkenyl, hydroxy or carboxy substituted alkyl or alkenyl, having from about 2 to about 20 carbon atoms, preferably from about 3 to about 14 carbon atoms. Other preferred synthetic esters conform to the following formula: 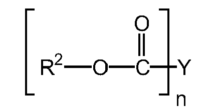 wherein R² is a C₈ to C₁₀ alkyl, alkenyl, hydroxyalkyl or hydroxyalkenyl group; preferably a saturated alkyl group, more preferably a saturated, linear, alkyl group; n and Y are as defined above. Specific non-limiting examples of suitable synthetic fatty esters for use in the compositions of the present invention include: P-43 (C₈-C₁₀ triester of trimethylolpropane), MCP-684 (tetraester of 3,3 diethanol-1,5 pentadiol), MCP 121 (C₈-C₁₀ diester of adipic acid), all of which are available from Mobil Chemical Company.

### Amino acids and protein hydrolysates

The hair conditioning agent can be protein hydrolysates and amino acids. Protein hydrolysates in terms of the invention are understood to be protein hydrolysates and/or amino acids and derivatives thereof. Derivatives are, for example, condensation products with fatty acids or cationically modified protein hydrolysates. Protein hydrolysates are product mixtures, which are obtained by decomposition (due to acidic, alkaline, or enzymatic catalysis) of proteins. The term protein hydrolysates is also understood to include total hydrolysates as well as individual amino acids and derivatives thereof as well as mixtures of various amino acids. Amino acids are, for example, alanine, arginine, asparagine, asparagine acid, cystine, glutamine, glutamine acid, glycine, histidine, isoleucine, leucine, lysine, methionine, phenylalanine, proline, serine, threonine, tryptophan, tyrosine, and valine. Furthermore, polymers constructed from amino acids and amino acid derivatives according to the present invention are included in the term protein hydrolysates. The latter includes, for example, polyalanine, polyasparagine, polyserine, etc. Other examples are L-alanyl-L-proline, polyglycine, glycyl-L-glutamine, or D/L-methionine-S-methylsulfonium chloride. β-amino acids and derivatives thereof such as β-alanine, anthranilic acid, or hippuric acid can also be used. The molar mass of the protein hydrolysates is between 75, the molar mass for glycine, and 200,000; the molar mass is preferably 75 to 50,000 and especially preferably 75 to 20,000 Dalton.
Protein hydrolysates of plant, animal, marine, or synthetic origin can be used. Animal protein hydrolysates are, for example, hydrolysates of elastin, collagen, keratin, silk, or lactoprotein, which can also be in the form of salts. According to the invention, the use of protein hydrolysates of plant origin, e.g. soy, almond, pea, potato, rice, and wheat protein hydrolysates as well as their condensation products with fatty acids are preferred. Even though the use of protein hydrolysates as such is preferred, if necessary, other obtained amino acid mixtures can be used in their place.
Suitable cationically modified protein hydrolysates are substance mixtures, which, for example, can be obtained by converting alkaline, acidic, or enzyme hydrolyzed proteins with glycidyl trialkyl ammonium salts or 3-halo-2-hydroxypropyl trialkyl ammonium salts. Proteins that are used as starting materials for the protein hydrolysates can be of plant or animal origin. Standard starting materials are, for example, keratin, collagen, elastin, soy protein, rice protein, lactoprotein, wheat protein, silk protein, or almond protein. The hydrolysis results in material mixtures with mole masses in the range of approx. 100 to approx. 50,000. Customary, mean mole masses are in the range of about 500 to about 1,000. It is advantageous if the cationically derived protein hydrolysates have one or two long C8 to C22 alkyl chains and two or one short C1 to C4 alkyl chain accordingly. Compounds containing one long alkyl chain are preferred. Cationic protein derivatives are known, for example, under the INCI designations Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein or Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, and Hydroxypropyltrimonium Hydrolyzed Vegetable Protein.

### Gel forming agents

In one embodiment of the invention, the fluid hair conditioning composition has the form of a gel or a cream gel and additionally contains at least one gel forming agent. The amount of gel forming agents is preferably from 0,05 to 30, more preferably from 0,2 to 20 and most preferably from 0,5 to 10% by weight based on the fluid composition. Suitable gel forming agents are for example one or a mixture of:
- synthetic polymer such as e.g. crosslinked polyacrylates;
- polymers on a natural basis, e.g. based on sclerotium gum; starch; gelatine; cellulose and cellulose derivatives such as carboxymethyl cellulose,hydroxyalkyl cellulose such as hydroxypropylcellulose or hydroxyethylcellulose, methylcelluose or hydroxyproyplmethylcellulose; microcrystalline cellulose; agar-agar; carrageenan, alginates, carouba gum, guar and guar derivatives such as alkylated and hydroxyalkylated guar; karaya gum; xanthan gum; dehydroxanthan; gum arabicum, pektin
- inorganic thickeners, e.g. hectorite, bentonite, metal silicates such as aluminium silicates or magnesium silicates.
   In particular, gel forming agents are:
   copolymers of at least one first monomer selected from acrylic acid and methacrylic acid and at least one second monomer selected from esters of acrylic acid and ethoxylated fatty alcohols; crosslinked polyacrylic acid; crosslinked copolymers of at least one first monomer selected from acrylic acid and methacrylic acid and at least one second monomer selected from esters of acrylic acid and C10 to C30 alcohols such as those with INCI-name Acrylates/C10-30 Alkyl Acrylate Crosspolymer having tradenames Pemulen™ TR-1, Pemulen™ TR-2, Carbopol™ 1342, Carbopol™ 1382, and Carbopol™ ETD 2020, all available from Noveon, Inc.; copolymers of at least one first monomer selected from acrylic acid and methacrylic acid and at least one second monomer selected from esters of itaconic acid and ethoxylated fatty alcohols; copolymers of at least one first monomer selected from acrylic acid and methacrylic acid and at least one second monomer selected from esters of itaconic acid and ethoxylated C10 to C30 alcohols and at least one third monomer selected from amino C 1 to C4-alkylacrylates; copolymers of two or more monomers selected from from acrylic acid, methacrylic acid, acrylic acid esters and methacrylic acid esters; copolymers of vinylpyrrolidone and ammonium acryloyl dimethyltaurate; copolymers of ammonium acryloyl dimethyltaurate and at least one monomer selected from esters of methacrylic acid and ethoxylated fatty alcohols; hydroxyethyl cellulose; hydroxypropyl cellulose; hydroxypropyl guar; glyceryl polyacrylate; glyceryl polymethacrylate; copolymers of styrene and at least one C2, C3 or C4-alkylene; gel forming polyurethanes; hydroxypropyl starch phosphate; polyacrylamide; copolymer of maleic acid anhydride and methylvinylether crosslinked wich decadiene; carob bean gum; guar gum; xanthan; dehydroxanthan; carrageenan; karaya gum; hydrolysed corn starch; copolymers of polyethylenoxide, fatty alcohols und saturated methylene diphenyldiisocyanate (e.g. PEG-150/stearyl alcohol/ SMDI copolymer).
   Gel forming polymers with acid groups are preferably neutralized up to 50 bis 100%. Non-limiting examples of neutralizing agents include primary or secondary organic amines, or inorganic bases such as ammonia, NaOH, KOH, ammonium hydroxide etc.. Preferred are amino alcohols with 1 to 10 carbon atoms and 1 to 3 hydroxy groups such as aminomethyl propanol (AMP), monethanolamine, diethanol amine, triethanolamine, tetrahydroxypropyl ethylendiamine, diisopropanolamine, tromethamine, and mixtures thereof.

### Solid carrier

The fluid hair conditioning composition is absorbed on a solid carrier. The solid carrier is preferably in the form of a powder consisting of a plurality of solid particles. The solid carrier according to the invention either consists of one solid compound or it is a composition or mixture of different solid compounds. Preferably it consists of a single, powdery solid compound. Powdery solids are for example zeolites, activated carbon, starch, modified starch, common salt, sugar, proteins, gelatin, titanium dioxide, highly disperse silicon dioxide, silicic acid, bentonite, lime, glutamate, phospholipids, cellulose and cellulose derivatives, polylactic acid, dextrin, kaolin, alginates, pectin, very finely ground plant components or a mixture of two or more of the above-mentioned substances each of which must be present in powder form.

The particle size of the powdery carrier is preferably at least 10 micrometer, more preferred at least 25 micrometer and preferably smaller or equal 500 micrometer, more preferred smaller or equal 200 micrometer, e.g. from 30 to 100 micrometer.

Although the absorption of the fluid hair conditioner composition on the solid carrier generally works with any solid, powdery material, it has been found that microcrystalline cellulose is best for hair care applications.

### Emulsifiers

Preferred embodiments of the invention include at least one emulsifier in the fluid composition in order to improve the washability of the composition from the hair and to further improve the perfomance benefits. The emulsifiers are preferably contained in an amount of from 0.5 to 20% by weight, especially preferably from 3 to 15% by weight, based on the fluid composition. In case the cationic hair conditioning surfactant also has sufficient emulsifying efficiency, the emulsifier can be the cationic hair conditioning surfactant itself. Otherwise it may be a non-cationic surfactant. Preferred emulsifiers are selected from the group of non-ionic surfactants.
Nonionic emulsifiers are for example
- alkoxylated fatty alcohols such as C8- to C30- or preferably C8- to C22-alcohols, alkoxylated fatty acids or alkoxylated fatty acid glycerides such as C 12 to C22-fatty acids, alkoxylated alkylphenols (e.g. alkyl groups with 8 to 15 carbon atoms); typical degrees of ethoxylation being from 2 to 100 or 4 to 30 and typical degrees of propoxylation being from 1 to 5;
- C8 to C30-, preferably C12- to C22-fatty acid glycerolmono- or diester, ethoxylated with from 1 to 30 mole ethylenoxide;
- Castor oil or hydrogenated castor oil ethoxylated with from 5 to 60 mole ethylenoxide;
- Fatty acid sugar mono- or diester, especially ester of sucrose with one or two C8- to C30 or C12 to C22-fatty acid, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate;
- ethoxylated sorbitan esters such as ester of sorbitan with one, two or three C8- to C22-fatty acid and a degree of ethoxylation of from 4 to 20;
- polyglyceryl fatty acid ester, especially of one, two or more C8- to C22-fatty acids with polyglycerol of preferably 2 to 20 glycerol units;
- alkylglucoside, alkyloligoglucoside or alkylpolyglucoside with C8- to C22-alkyl groups, e.g. Decyl Glucoside oder Lauryl Glucoside.

### Optional ingredients

The products according to the invention can also contain conventional cosmetic additives usually used in hair treatment compositions in addition to the above-mentioned ingredients, e.g. fragrances and perfume oils in an amount of up to 2% by weight, preferably from 0.01 to 1% by weight; preservatives such as for example parabenes, phenoxetol, iodopropynyl carbamate, parahydroxybenzoic acid ester, benzoic acid, salicylic acid, sorbic acid, mandelic acid, polyhexamethylene biguanidine hydrochloride or isothiazoline based compounds in an amount of for example up to 2% by weight, preferably 0.01 to 1% weight; buffer substances, such as sodium citrate or sodium phosphate, in an amount of 0.1 to 1% by weight; further hair care substances, such as e.g. moisturizer, vitamins or plant extracts in an amount of for example 0.01 to 5%, preferably 0.1 to 4% by weight; light protective agents, antioxidants, radical-trapping agents, antidandruff agents in an amount of 0.01 to 2% by weight.

### METHOD OF MAKING

The fluid hair conditioning composition can be made by conventional formulation and mixing techniques generally known to a person skilled in the art. The non-fluid hair conditioning product according to the invention which is a combination of a fluid composition absorbed on a solid carrier, can be made by pulverizing a fluid hair conditioning composition (A) on a non-fluid, powdery, solid carrier (B), wherein said fluid hair conditioning composition (A) contains at least one hair care agent dissolved or dispersed in at least one solvent which is liquid at room temperature (25 °C), and wherein said solid carrier (B) is solid at room temperature (25 °C). Preferred hair care agents are the hair conditioning substances described above. The term "pulverizing a fluid composition on a non-fluid carrier" as used herein, means a process of making a non-fluid (e.g. powdery) end product from a fluid (e.g. liquid or gel) composition and a non-fluid (e.g. solid) carrier. The fluid is absorbed on the carrier. The term "absorbed" as used herein, means that either the surface of a non-fluid (e.g. solid) carrier particle is partly or completely coated or covered by the fluid or that the fluid is contained in cavities or pores of the carrier particle.

A general description of a method for producing a powder product from a liquid substance and a solid, powdery carrier by using compressed gases is described in WO 99/17868. The products according to the invention can be made by this method using liquid, gel-form or cream-form hair conditioning compositions as the fluid substance and a suitable solid carrier. This method is also known as CPF-technology (Concentrated Powder Form) or as cryogenic high-pressure spray technology. In one embodiment of the invention the non-fluid hair conditioning product is a product made by first dissolving a gas in a fluid hair conditioning composition at high pressure, then expanding the liquid/ gas solution, wherein a solid carrier is added in solid form either before, or during or shortly after said expansion. This process for producing a powdery product from a composition that is fluid at room temperature, has the steps:
• providing, in a pressure vessel, the fluid composition to be pulverized,
• dissolving a gas (e.g. carbon dioxide) in the fluid composition under elevated pressure (e.g. 100 to 250 bar),
• conducting the fluid/gas solution out of the pressure vessel to an expansion element, and
• passing the fluid/gas solution through the expansion element for rapid expansion of the solution,
wherein a solid, powdery carrier is admixed to the fluid upstream of the expansion element, in the expansion element or downstream, in particular just downstream, of the expansion element. The obtained non-solid product can be separated from gas and remaining liquids by conventional methods, e.g. sedimentation, filtration, cyclone or electrical field. The expansion process taking place during passage of the liquid/gas solution through the expansion element can be carried out in such a manner that the temperature roughly attains or falls below the solidification temperature of the fluid composition. The gas can be dissolved in the fluid composition until the fluid composition is essentially saturated with the gas. Suitable gases are e.g. carbon dioxide, hydrocarbons, in particular methane, ethane, propane, butane, ethene, propene, or a halogenated hydrocarbon, an ether, an inert gas, in particular nitrogen, helium or argon, a gaseous oxide, in particular dinitrogen oxide or sulphur dioxide, ammonia, or a mixture of two or more of the above-mentioned gases. Most preferred is carbon dioxide. The elevated pressure under which the gas is dissolved in the fluid composition can be in the range from 5 bar to 800 bar, preferably in the range from 10 bar to 350 bar, and particularly preferably in the range from 20 bar to 250 bar. The gas can be mixed with the fluid composition, e.g. by a static mixer, by shaking or rolling the pressure vessel, by stirring the solution forming in the pressure vessel, by recirculating the liquid phase and/or gas phase present in the pressure vessel, or by a combination of two or more of the above mentioned procedures. The amount of solid carrier, based on the total amount of fluid hair conditioning composition and solid carrier, can be e.g. between 10 and 70% by weight, preferably between 20 and 60% by weight, and particularly preferably between 30 and 45% by weight. The expansion element can be a nozzle, a diffuser, a capillary, an orifice plate, a valve or a combination of the said expansion elements. The solid carrier can be fed to the mass stream, which is exiting from the expansion element, in the area of the outlet point. The fluid/gas solution can be expanded into a spray tower. Gas can be additionally fed into the fluid/gas solution between the pressure vessel and the expansion element, in particular just upstream of the expansion point. The fluid/gas solution and additionally supplied gas can be expanded together with one another in the expansion element by means of a two-component nozzle. Additional gas can also be fed together with the feed of the solid powdery carrier to the fluid composition. More details of the process are described in WO 99/17868.

### METHOD OF USE

An embodiment of the invention is a method of hair conditioning, said method comprising the steps of:
a) providing a non-fluid hair conditioning product according to the invention described above,
b) mixing the non-fluid hair conditioning product with water prior to use,
c) applying said mixture of non-fluid hair conditioning product and water to the hair, and
d) rinsing the hair.
The rinsing of the hair is typically done with water and can be done immediately or after a residence time of e.g. from 30 seconds or from 1 minute up to 2, 5, 10 or even 30 min, if necessary depending on the type of hair, product type and intensity of desired conditioning effect. In one aspect of the invention, heat can be applied during the residence time, i.e. after application of the product to the hair and before rinsing. Conventional heating devices such as blow driers or infrared devices can be used for applying the heat. Typically, the temperature can be above 30°C, preferably above 40°C and up to 70°C or 80°C.

Such method generally involves application of an effective amount of the product to dry, slightly damp, or wet hair, preferably on wet hair after a hair wash. By "effective amount" is meant an amount sufficient to provide the hair conditioning benefits desired considering the length and texture of the hair. In general, from about 0.5 g to about 50 g of product will be applied to the hair, depending upon the particular product formulation, length of hair, and hair type. The mixing ratio of powdery product to water can vary due to the product consistency and viscosity desired or preferred by the user. The weight ratio can be e.g. from 1:0.5 to 1:5 or from 1:1 to 1:2.

Another application possibility is to add the powdery hair conditioner of the invention to a conventional, non-powdery hair conditioning product prior to its application on hair. Further applications possibilities are uses in connection with hair coloring products and methods. The powdery hair conditioner of the invention can be mixed with a conventional hair coloring product prior to its use or can be applied to the hair after a coloring process. The hair coloring product can be based on direct dyes, oxidation dyes or a mixture of both.

Products according to the invention of the type of the exemplary compositions described below will have similar hair conditioning effects as conventional hair conditioning products but have benefits over conventional hair conditioners in giving the user, e.g. a hair dresser, a greater flexibilty to treat different type of hairs, or to treat different parts of the hair differently or to satisfy individual needs, habits or preferences, e.g. for product textures. Highly damaged hair can be treated with a pulverized product/water mixture using less amounts of water and thus a higher concentration of active ingredients than will be used for less damaged hair. Or the hair tips are treated specifically with a higher concentrated pulverized product/water mixture than the rest of the hair.
This simplifies and reduces the number of products a hair dresser has to have on stock to satisfy a greater number of different demands due to the diversity of the hair types of the customers. Also, the hair dresser or consumer can individually adjust the texture and viscosity of the product/water mixture according to her specific preference and working technique.

### EXAMPLES

The compositions illustrated in the following examples illustrate specific embodiments of the pulverized, non-fluid hair conditioning products of the present invention, but are not intended to be limiting thereof. Other modifications can be undertaken by the skilled artisan without departing from the spirit and scope of this invention.

The fluid conditioning compositions illustrated in the following examples are prepared by conventional formulation and mixing methods. The final products comprising the liquid conditioning composition absorbed on the solid carrier is prepared by the method described in WO 99/17868 using carbon dioxide as gas for dissolving and subsequent expanding. All exemplified amounts are listed as weight percents and exclude minor materials such as diluents, preservatives, colour solutions, imagery ingredients, botanicals, and so forth, unless otherwise specified. If a trade name is mentioned as ingredient and the respective product is itself a mixture (e.g. a solution, emulsion, dispersion etc.), then the exemplified amount relates to this mixture, unless otherwise specified.

### Example 1

### Fluid conditioning composition: Hair conditioning cream treatment

| | |
|---|---|
| 3,2 | Genamin® KDMP (80% behenyltrimethylammonium chloride in isoproanol) |
| 2,5 | Cetearyl alcohol |
| 1,5 | Dow Coming 939 Emulsion ¹⁾ |
| 0,5 | Hydroxypropyl starch phosphate |
| 1,0 | Abil Quat® 3272 (50% Quaternium-80 in propylene glycol) |
| 0,5 | D-panthenol |
| 0,1 | Buttermilk powder |
| 0,05 | sea salt |
| 0,05 | Tocopheryl acetate |
| 0,05 | Apricot kernel oil |
| 0,05 | Vitamincomplex A, E, F, H' of Crodarom (4,5% in Polysorbate-20, water) |
| 0,05 | Grape leaf extract |
| q.s. | Fragrance, preservatives |
| Ad 100 | Water |

| | |
|---|---|
| ¹⁾ 33% Amodimethicone, 3% Cyclomethicone, 3%Trideceth-12, 0,6% Cetrimonium chloride in water | |

A pumpable, white cream emulsion with a viscosity of about 1200 mPa s (MV-DIN, 12,9 s⁻¹ , 25°C after 24 hours) and a pH in the range of 6,0 to 7,5 is produced by conventional mixing and emulsification procedure.

### Powdery, solid carrier:

1a) Avicel PC101, microcrystalline cellulose, 50 micron
1b) Vivapur 101, microcrystalline cellulose, 50 micron
1c) Vivapur 102, microcrystalline cellulose, 90 micron

Pulverized hair conditioning products are produced by absorbing the fluid conditioning composition on one of the powdery solid carrier a, b or c according to the method described in WO 99/17868 using carbon dioxide as gas. The following weight ratios are used:
- Ex. 1.1:: 28,7% Fluid / 71,3% microcrystalline cellulose carrier
- Ex. 1.2:: 34,9% Fluid / 65,1 % microcrystalline cellulose carrier
- Ex. 1.3:: 36,2% Fluid / 63,8% microcrystalline cellulose carrier
- Ex. 1.4:: 40,2% Fluid / 59,8% microcrystalline cellulose carrier
- Ex. 1.5:: 43,2% Fluid / 56,8% microcrystalline cellulose carrier

The pulverized hair conditioning products are non-fluid, slightly agglomerated powdery materials. The powdery products are mixed with water immediately before use on hair. The powdery products provide the hairdresser to provide a customized treatment for her client. The treatment can be dosed depending on the specific hair damage and hair structure. The hair dresser can choose the amount of water to achieve the desired viscosity and consistency.

For sensory tests, the powder was mixed with water in a weight ratio of 1: 1,5. The powdery products are easy to disperse with water and form homogeneous, creamy conditioner with a smooth to slightly grainy feeling. The distribution and consistency differs from conventional hair conditioning treatments and from the non-pulverized fluid hair conditioning cream treatment as reference.

The application technical properties have been assessed by hair dresser professionals. The performance in wet and dry hair was rated nearly similar to the reference. New applications can be provided by the pulverized treatments. For example, the pulverized treatment can be applied directly to wet hair by a shaker can.

### Example 2

### Fluid conditioning composition: Nourishing hair mask

| | |
|---|---|
| 6,0 | Cetylalcohol |
| 0,05 | Cholesterol |
| 1,2 | Genamin® KDMP (80% behenyltrimethylammonium chloride in isoproanol) |
| 4,0 | Mineral oil (paraffinum perliquidum DAB) |
| 2,5 | Isopropyl myristate |
| 2,0 | Lamesoft® PO 65 ¹⁾ |
| 1,0 | Cetyltrimethylammonium chloride |
| 1,0 | Dow Coming 949 Cationic Emulsion ²⁾ |
| 0,1 | Citric acid |
| q.s. | Fragrance, preservative |
| Ad 100 | Water |

| | |
|---|---|
| ¹⁾mixture of 33% glyceryl oleate and 33% coco-glucoside in water ²⁾ 33% Amodimethicone, 2% Cyclotetrasiloxane, 2%Trideceth-12, 2% Cetrimonium chloride in water | |

A white, homogeneous emulsion with a viscosity in the range of 6000 to 7000 mPa s (SV-DIN, 12,9 s⁻¹, 25°C) and a pH in the range of 2,7 to 3,7 is produced by conventional mixing and emulsification procedure.

### Powdery, solid carrier:

2a) Avicel PC101, microcrystalline cellulose, 50 micron; moisture content 5%; powder density 0,26-0,3 g/cm³
2b) Vivapur 101, microcrystalline cellulose, 50 micron; moisture content 5%; powder density 0,29 g/cm³
2c) Vivapur 102, microcrystalline cellulose, 90 micron; moisture content 5%; powder density 0,31 g/cm³
2d) Dry Flo Plus, Aluminium Starch Octenylsuccinate; moisture content 14%
2e) Remy FG KA, Oryza Sativa (Rice) Starch; < 75 micron; moisture content 14%

Pulverized hair conditioning products are produced by absorbing the fluid conditioning composition on one of the powdery solid carrier a, b or c according to the method described in WO 99/17868 using carbon dioxide as gas. The following weight ratios are used:
- Ex. 2.1:: 23,1% Fluid / 76,9% carrier 2e
- Ex. 2.2:: 8,5% Fluid / 91,5% carrier 2f
- Ex. 2.3:: 31,2% Fluid / 78,8% carrier 2a
- Ex. 2.4:: 47,0% Fluid / 53,0% carrier 2b
- Ex. 2.5:: 44,4% Fluid / 55,6% carrier 2c

The pulverized hair conditioning products of ex. 2.3, 2.4 and 2.5 are non-fluid, free flowing powdery materials. Examples 2.1 and 2.2 showed significant agglomeration. The powdery products are mixed with water immediately before use on hair. The powdery products provide the hairdresser to provide a customized treatment for her client. The treatment can be dosed depending on the specific hair damage and hair structure. The hair dresser can choose the amount of water to achieve the desired viscosity and consistency.

The application technical properties have been assessed by hair dresser professionals. The conditioning effects for ex. 2.3, 2.4 and 2.5 were rated nearly similar to the not pulverized nourishing hair mask as reference with the additional advantage that the hair felt cleaner without heaviness when treated with the pulverized hair mask/water mixture. Examples 2.3, 2.4 and 2.5 with microcrystalline cellulose carrier are preferred over examples 2.1 and 2.2 with starch and starch derivative carrier because of better free flow characteristics, easier metering and stirring in water, better distribution on hair and easier rinsing from the hair after application. Example 2.4 with the highest loading is preferred.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A pulverized, non-fluid hair conditioning product comprising a fluid hair conditioning composition which is absorbed on a solid carrier, wherein the fluid hair conditioning composition contains at least one hair conditioning agent, selected from the group consisting of hair conditioning surfactants, hair conditioning polymers, hair conditioning silicones, fatty alcohols, oils, panthenol, amino acids, panthenyl ethyl ether, sorbitol, betaine, creatine and protein hydrolysates; and
wherein said carrier is a non-waxy material which is solid at room temperature (25 °C).

2. A non-fluid hair conditioning product according to the preceding claim, wherein the pulverized, non-fluid hair conditioning product is made by first dissolving a gas in said fluid hair conditioning composition at high pressure, then expanding the liquid/gas solution, wherein said solid carrier is added either before, or during or shortly after said expansion.

3. A non-fluid hair conditioning product according to any of the preceding claims, wherein the carrier is loaded with the fluid hair conditioning composition in an amount of at least 20% by weight based on the total amount of product.

4. A non-fluid hair conditioning product according to any of the preceding claims, wherein the hair conditioning surfactants are selected from cationic surfactants of general formula
N⁽⁺⁾R¹R²R³R⁴ X⁽⁻⁾
wherein R1 to R4 independently from one another stand for aliphatic groups, aromatic groups, alkoxy groups, polyoxyalkylene groups, alkylamido groups, hydroxyalkyl groups, aryl groups or alkaryl groups with 1 to 22 carbon atoms, wherein at least one residue has at least 6 carbon atoms and X⁻ represents an anion.

5. A non-fluid hair conditioning product according to any of the preceding claims, wherein the, hair conditioning polymers are cationic or zwitterionic polymers selected from the group consisting of cationic cellulose derivatives from hydroxyethyl cellulose and diallyl dimethyl ammonium chloride; cationic cellulose derivatives from hydroxyethyl cellulose and epoxide substituted with trimethyl ammonium; poly(dimethyldiallyl ammonium chloride); copolymers from acrylamide and dimethyldiallyl ammonium chloride; quaternary ammonium polymers, formed by the reaction of diethylsulfate with a copolymer from vinyl pyrrolidone and dimethylaminoethyl methacrylate; quaternary ammonium polymers from methylvinylimidazolium chloride and vinyl pyrrolidone; Polyquaternium-35; polymer from trimethyl ammonium ethyl methacrylate chloride; Polyquaternium-57; dimethylpolysiloxane terminally substituted with quaternary ammonium groups; copolymer from vinyl pyrrolidone, dimethylaminopropyl methacrylamide, and methacryloylamino propyl lauryl dimethyl ammonium chloride; chitosan and salts thereof; hydroxyalkyl chitosans and salts thereof; alkyl hydroxyalkyl chitosans and salts thereof; N-hydroxyalkyl chitosan alkyl ether; copolymer from vinyl caprolactam, vinyl pyrrolidone, and dimethylaminoethyl methacrylate; copolymers from vinyl pyrrolidone and dimethylaminoethyl methacrylate, copolymers from vinyl pyrrolidone, vinyl caprolactam, and dimethylaminopropylacrylamide; poly- or oligoesters, constructed from at least one first type of monomer, which is selected from hydroxycarboxylic acid substituted with at least one quaternary ammonium group, copolymers from lauryl acrylate, stearyl acrylate, ethylamine oxide methacrylate, and at least one monomer selected from acrylic acid, methacrylic acid, acrylic acid esters, and methacrylic acid esters; copolymers from methacryloyl ethyl betaine and at least one monomer selected from methacrylic acid and methacrylic acid esters; copolymers from acrylic acid, methylacrylate, and methacrylamide propyl trimethylammonium chloride; oligomers or polymers that can be produced from quaternary crotonic betaines or quaternary crotonic betaine esters.

6. A non-fluid hair conditioning product according to any of the preceding claims, wherein the, hair conditioning silicones are selected from the group consisting of silicone oils, amino silicones, cationic silicones, silicone gums, high refractive silicones and silicone resins.

7. A non-fluid hair conditioning product according to any preceding claim, wherein the solid carrier is microcrystalline cellulose.

8. A non-fluid hair conditioning product according to any preceding claim, wherein the fluid hair conditioning composition is an aqueous emulsion containing
(A) 0,01 to 20 wt.% based on the emulsion of at least one hair conditioning agent selected from the group consisting of hair conditioning cationic surfactants, hair conditioning cationic polymers and hair conditioning silicones, and
(B) 0,5 to 20 wt.% based on the emulsion of at least one emulsifying surfactant, and
(C) at least one oily or fatty compound; and
(D) water.

9. Method of hair conditioning, wherein
- a non-fluid hair conditioning product according to any preceding claim is provided,
- the non-fluid hair conditioning product is mixed with water prior to use,
- the mixture of said non-fluid hair conditioning product with water is applied to the hair; and
- the hair is rinsed.

10. Use of a product according to any of claims 1 to 8 for conditioning human hair.
